# EUROPEAN PATENT APPLICATION

(11) **EP 4 768 916 A1**
(43) Date of publication of application: **01.07.2026**
(21) Application number: 24926621.4
(22) Date of filing: 15.07.2024
(51) Int. Cl.: G01N 33/558, G01N 33/543

(54) **HAZARD FACTOR TEST CASSETTE AND METHOD**

(30) Priority: 01.03.2024 CN 202410235002
(71) Applicant: Institute of Process Engineering, Chinese Academy of Sciences, Beijing 100190 (CN)
(72) Inventor: ZHOU, Lei, Beijing 100190 (CN); SUN, Chongsi, Beijing 100190 (CN); HU, Qiushi, Beijing 100190 (CN)
(74) Representative: Lavoix
(86) International application number: PCT/CN2024/105577
(87) International publication number: WO 2025/179752

(57) **Abstract**

A hazard factor test cassette and method. The hazard factor test cassette comprises: flow guide paths, the plurality of flow guide paths being respectively and independently communicated with a sample injection port(1) and flow guide platforms(2); each flow guide path comprises a long-distance flow guide path(3) and a short-distance flow guide path(4); the length for the long-distance flow guide paths(3) connecting the sample injection port(1) and the flow guide platforms(2) is greater than that of the short-distance flow guide paths(4); test strips for testing small-size targets are provided on the flow guide platforms(2) corresponding to the long-distance flow guide paths(3); and test strips for testing large-size targets are provided on the flow guide platforms(2) corresponding to the short-distance flow guide paths(4).The hazard factor test method uses the hazard factor test cassette for test, and comprises sequentially arranging toxin type test strips, virus type test strips and bacterium type test strips on paths from the long-distance flow guide paths(3) to the short-distance flow guide paths(4) among the short-distance flow guide paths(4) and the long-distance flow guide paths(3).

## Description

### CROSS-REFERENCE TO RELATED APPLICATIONS

The present application claims the priority of the Chinese Patent Application No. 202410235002.2, filed with the China National Intellectual Property Administration on March 1, 2024, entitled "HAZARD FACTOR TEST CASSETTE AND METHOD", the entire contents of which are incorporated herein by reference.

### TECHNICAL FIELD

The present application relates to the technical field of immunodiagnostics, and more particularly, to a hazard factor test cassette and method based on bio-probe multi-channel immunochromatography.

### BACKGROUND ART

The hazard factor test cassette adopts immunochromatography technology, which is a relatively mature on-site rapid detection technology. Multi-channel immunochromatographic test discs have been disclosed in the prior art. In the existing multi-channel immunochromatographic test discs and the prior patents of the applicant, for example, the patent application with publication number CN101261270A discloses that the unique multi-stage design and flow-guide pad of the test disc realize the uniform distribution of sample solution among the test strips in each channel and the subsequent synchronous chromatography of the sample solution, thereby enabling the simultaneous detection of multiple targets. However, some problems still exist: due to the lack of strict flow channel distinction from sample addition to the chromatography of multiple target detection test strips, the sample solution may reflux after contacting the sample pad and conjugate pad of the test strips, and cross-channel flow disturbance may occur between adjacent channels, which affects the detection sensitivity and specificity; the current structure can only achieve synchronous chromatography of the sample solution on the multi-channel detection test strips, rather than the synchronous chromatography of the target analytes to be detected therein.

Due to the wide variety of biological hazard factors and the different sizes of different types of biological hazard factors, the biological hazard factor test cassette in the prior art lack designs for preventing cross-channel flow disturbance and ensuring the synchronization of chromatographic detection for multiple types of targets, such as bacteria, viruses, and toxins. For example, as particles dispersed and suspended in a solvent, bacteria, viruses, and toxins have differences in size and resistance. When bacteria, viruses, and toxins pass through the test strips with microporous structures (such as flow-guide pads, sample pads, conjugate pads, and analytical membranes) along with the universal sample treatment solution, the larger-sized bacterial particles encounter the greatest resistance and exhibit the slowest chromatography, while the smallest-sized toxins encounter the least resistance and exhibit the fastest chromatography. To achieve synchronous chromatography of biological hazard factor targets of different sizes on the same detection test cassette and improve detection sensitivity, detection test strips for different targets are assigned to different guide platforms and flow guide paths of different lengths according to the sizes of the respective targets.

### SUMMARY

Therefore, the technical problem to be solved by the present application lies in overcoming the lack of biological hazard factor detection technology in the prior art that enables synchronous chromatography of multiple types of targets (such as bacteria, viruses, and toxins) in a single test. To this end, the present application provides a hazard factor test cassette, which is a hazard factor test cassette based on bio-probe multi-channel immunochromatography, it includes:
an outer shell;
a sample injection port for loading a sample;
flow guide platforms, a number of the flow guide platforms being 2 to 20, and the flow guide platforms being configured to receive individual detection test strips; and
flow guide paths, a number of the flow guide paths being 2 to 20, each of the flow guide paths and a flow-guide pad independently communicating with the sample injection port and one of the flow guide platforms, respectively; the flow guide paths comprising: long-distance flow guide paths and short-distance flow guide paths; a length of connection between the sample injection port and the guide platforms via the long-distance flow guide paths being greater than that via the short-distance flow guide paths; the detection test strips for detecting small-size targets are disposed on the flow guide platforms corresponding to the long-distance flow guide paths, and the detection test strips for detecting large-size targets are disposed on the flow guide platforms corresponding to the short-distance flow guide paths.

Optionally, the outer shell includes: an upper shell and a lower shell assembled and connected with each other.
the flow guide paths comprise: flow-guide grooves disposed on one of the upper shell and the lower shell, and flow-guide sheets disposed on the other of the upper shell and the lower shell; and
in an assembled state of the outer shell, the flow-guide sheets are inserted and fixed in the flow-guide grooves.

Optionally, the flow-guide sheets and the flow-guide grooves are inserted and connected in an interference fit manner.

Optionally, the hazard factor test cassette further comprising: rivet structures disposed on one of the upper shell and the lower shell, and annular groove structures disposed on the other of the upper shell and the lower shell; and
the rivet structures and the annular groove structures are inserted and connected in a matching manner to fix the upper shell and the lower shell together.

Optionally, the rivet structures and the annular groove structures that are connected in the matching manner are disposed on a side of the outer shell away from the flow guide paths.

Optionally, a plurality of the flow guide paths are disposed in a width direction of the outer shell;
the sample injection port is an elliptical structure extending along the width direction of the outer shell, and each of the long-distance flow guide paths and/or the short-distance flow guide paths is provided with: a smooth curved section extending toward the sample injection port, and a connecting section extending toward the flow guide platforms.

Optionally, the hazard factor test cassette further including:
a wide-vision detection window being configured to observe chromatographic states and final detection results of the detection test strips.

Optionally, a number of the wide-vision detection window on the outer shell is one, the wide-vision detection window is disposed corresponding to a central axis of the sample injection port; the long-distance flow guide paths and the short-distance flow guide paths are both disposed opposite to the sample injection port; or
a number of the wide-vision detection windows on the outer shell is two, the two wide-vision detection windows are symmetrically disposed on both sides of the sample injection port; and the long-distance flow guide paths and the short-distance flow guide paths are disposed on both sides of the sample injection port.

Optionally, the lower shell is provided with baffles for separating the detection test strips, and the upper shell is further provided with fixing columns for positioning the detection test strips.

A hazard factor detection method for using the hazard factor test cassette as mentioned above, wherein the method includes:
step S1: preparing detection test strips for virulent biological hazard factors and preparing a universal sample treatment solution;
step S2: placing the detection test strips for identifying different types of the virulent biological hazard factors on different flow guide platforms of a multi-channel test cassettes, placing a flow-guide pad at a sample loading position, and covering and pressing an upper cover tightly;
step S3: adding 1 mL to 3 mL of a sample prepared with the universal sample treatment solution into the sample injection port, recording a chromatographic time to determine a detection time;
step S4: inserting a test strip end of a test cassette into a detector to a fixed position;
step S5: starting the detector to perform quantitative determination on the test strips through the wide-vision detection window, and the detector displays a determination result;
step S6: adding a fully negative sample into the sample injection port of the test cassette, and reading a result on the detector, wherein each of the detection test strips is provided with an obvious quality control peak and an extremely low detection peak, and a ratio of the detection peak value to the quality control peak value presents a negative result; and
step S7: adding a to-be-detected sample prepared with the universal sample treatment solution into the sample injection port of the test cassette, reading the result on the detector, and determining a result of a certain virulent biological hazard factor in the sample by regressing the ratio of the detection peak value to the quality control peak value with a standard curve built in the detector;
wherein, in step S2:
disposing the detection test strips for detecting large-size targets on the short-distance flow guide paths, and disposing the detection test strips for detecting small-size targets on the long-distance flow guide paths;
wherein, toxin-based detection test strips, virus-based detection test strips and bacteria-based detection test strips are sequentially disposed on the channels from long distances to short distances among the short-distance flow guide paths and the long-distance flow guide paths.

The technical solution of the present application has the following advantages:
1. The hazard factor test cassette provided by the present application includes: an outer shell; a sample injection port for loading a sample; flow guide platforms, the number of the flow guide platforms being 2 to 20, and the flow guide platforms being configured to receive individual detection test strips;
flow guide paths, the number of which ranges from 2 to 20, each independently communicating with the sample injection port and one of the guide platforms respectively; the flow guide paths include long-distance flow guide paths and short-distance flow guide paths; the length of the connection between the sample injection port and the flow guide platforms via the long-distance flow guide paths is greater than that via the short-distance flow guide paths; the detection test strips for detecting small-size targets are disposed on the flow guide platforms corresponding to the long-distance flow guide paths, and the detection test strips for detecting large-size targets are disposed on the flow guide platforms corresponding to the short-distance flow guide paths.

In the present application, the flow guide paths are divided into long-distance flow guide paths and short-distance flow guide paths. Meanwhile, the detection test strips for detecting small-size targets are disposed on the long-distance flow guide paths, and the detection test strips for detecting large-size targets are disposed on the short-distance flow guide paths. This enables the multi-channel test cassette to be compatible with the detection of three types of targets, namely bacteria, viruses, and toxins. Based on the inherent size and resistance characteristics of bacteria, viruses, and toxins, relying on this flow-guide fixing structure and test strip distribution method, the chromatographic progress of different types of targets to be detected (such as bacteria, viruses, and toxins) on their respective detection test strips is synchronized. This achieves consistent target chromatography and capture time across multiple channels, thereby ensuring the highest detection result sensitivity of the multi-channel system.

For bacteria and toxins dispersed in the sample treatment solution, their resistance during the chromatographic process varies due to differences in their own particle sizes. The chromatographic process occurs in the flow-guide pad and all parts of the detection test strips that the sample flows through after being added to the sample injection port. The targets flow through the aforementioned porous structural materials along with the sample treatment solution and undergo important reactions such as specific recognition and specific capture. The chromatography and reaction time of the targets in the sample are directly related to the detection sensitivity. Larger-sized bacteria encounter greater resistance during chromatography, resulting in slower chromatography of bacterial targets, while smaller-sized toxins encounter less resistance during chromatography, leading to faster chromatography of toxin targets. Therefore, in the present application, the bacteria-based detection test strips are disposed on the short-distance flow guide paths, and the toxin-based detection test strips are disposed on the long-distance flow guide paths.

2. The hazard factor test cassette provided by the present application, wherein the outer shell includes an upper shell and a lower shell assembled and connected with each other; the flow guide paths include flow-guide grooves disposed on one of the upper shell and the lower shell, and flow-guide sheets disposed on the other of the upper shell and the lower shell; in an assembled state of the outer shell, the flow-guide sheets are inserted and fixed in the flow-guide grooves.

In the present application, the flow guide paths are formed by the aforementioned assembled and inserted flow-guide grooves and flow-guide sheets. The cooperatively inserted and connected flow-guide grooves and flow-guide sheets effectively prevent the sample treatment solution from flowing out through the gaps between different flow guide paths between the upper shell and the lower shell. This not only can effectively block the cross-channel flow of the sample treatment solution between different flow guide paths, ensure the uniform distribution of the sample treatment solution in different flow guide paths, and guarantee the correctness and sensitivity of the detection results of different channels, but also the aforementioned flow-guide grooves and flow-guide sheets can effectively fix the upper shell and the lower shell of the test cassette together. Therefore, the test cassette provided by the present application does not need to be provided with a clamping structure for fixing the upper shell and the lower shell together near the flow guide paths, thereby reducing the size of the test cassette and realizing the miniaturization of the test cassette.

3. The hazard factor test cassette provided by the present application, wherein the flow-guide sheets and the flow-guide grooves are inserted and connected in an interference fit manner. Fixing the flow-guide sheets and the flow-guide grooves together through the interference fit can effectively further prevent the problems of cross-channel flow between adjacent flow guide paths and flow disturbance of reflux reagent between adjacent flow guide paths. Meanwhile, it fixes the upper shell and the lower shell more firmly together.

4. The hazard factor test cassette provided by the present application further includes: rivet structures disposed on one of the upper shell and the lower shell, and annular groove structures disposed on the other of the upper shell and the lower shell; the rivet structures and the annular groove structures are inserted and connected in a matching manner to fix the upper shell and the lower shell together.

In the present application, the above-mentioned mutually matching rivet structures and annular groove structures can effectively fix the upper shell and the lower shell together, while ensuring the firmness of the connection and cooperation between the flow-guide sheets and the flow-guide grooves.

5. The hazard factor test cassette provided by the present application further includes: a wide-vision detection window, through which operators can conveniently observe the chromatographic states and final detection results of the detection test strips.

6. The hazard factor test cassette provided by the present application, wherein the lower shell is provided with baffles for separating the detection test strips, and the upper shell is further provided with fixing columns for positioning the detection test strips. In the present application, the baffles and fixing columns can effectively limit and fix the detection test strips in each of the flow guide paths, thereby avoiding displacement of the detection test strips which would affect the detection speed and the accuracy of the detection results.

7. The hazard factor test method provided by the present application is used with the aforementioned hazard factor test cassette. By disposing the detection test strips for detecting large-size targets on the short-distance flow guide paths and the detection test strips for detecting small-size targets on the long-distance flow guide paths, the above arrangement can effectively ensure that the detection result sensitivity of multiple channels is relatively high, while guaranteeing the detection speed and detection accuracy.

### BRIEF DESCRIPTION OF THE DRAWINGS

To more clearly illustrate the specific implementations of the present application or the technical solutions in the prior art, the accompanying drawings required for describing the specific implementations or the prior art will be briefly introduced below. Obviously, the accompanying drawings in the following description are some implementations of the present application, and for those of ordinary skill in the art, other accompanying drawings can also be obtained based on these drawings without exerting creative labor.
FIG. 1 is a three-dimensional structural schematic diagram of a ten-channel lower shell with one wide-vision detection window in embodiment 1 provided by the present application;
FIG. 2 is a three-dimensional structural schematic diagram of a ten-channel upper shell with one wide-vision detection window in embodiment 1 provided by the present application;
FIG. 3 is a schematic diagram of the relative positions of the smooth curved section and the connecting section in the flow-guide channel provided by the present application;
FIG. 4 is a three-dimensional structural schematic diagram of a sixteen-channel lower shell with one wide-vision detection window in embodiment 2 provided by the present application; and
FIG. 5 is a three-dimensional structural schematic diagram of a sixteen-channel upper shell with one wide-vision detection window in embodiment 2 provided by the present application.

### Explanation of reference numerals:

1-Sample injection port; 2-flow guide platforms; 3-Long-distance flow guide path; 4-Short-distance flow guide path; 5-Upper shell; 6-Lower shell; 7-Flow-guide groove; 8-Flow-guide sheet; 9-Rivet structure; 10-Annular groove structure; 11-Wide-vision detection window; 12-Smooth curved section; 13-Connecting section; 14-Baffle; 15-Fixing column.

### DETAILED DESCRIPTIONS OF THE EMBODIMENTS

The technical solutions of the present application will be clearly and completely described below in conjunction with the accompanying drawings. Obviously, the described embodiments are part of the embodiments of the present application, rather than all of the embodiments. Based on the embodiments in the present application, all other embodiments obtained by those of ordinary skill in the art without making creative labor shall fall within the protection scope of the present application.

### Embodiment 1

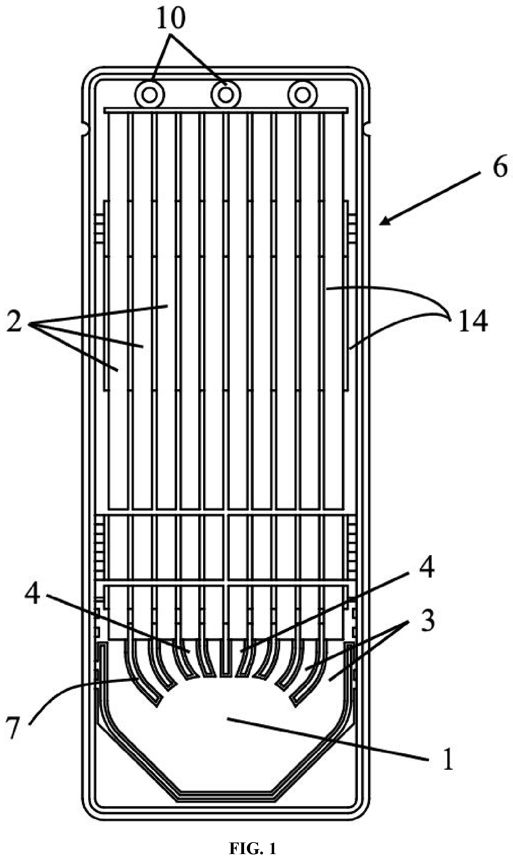

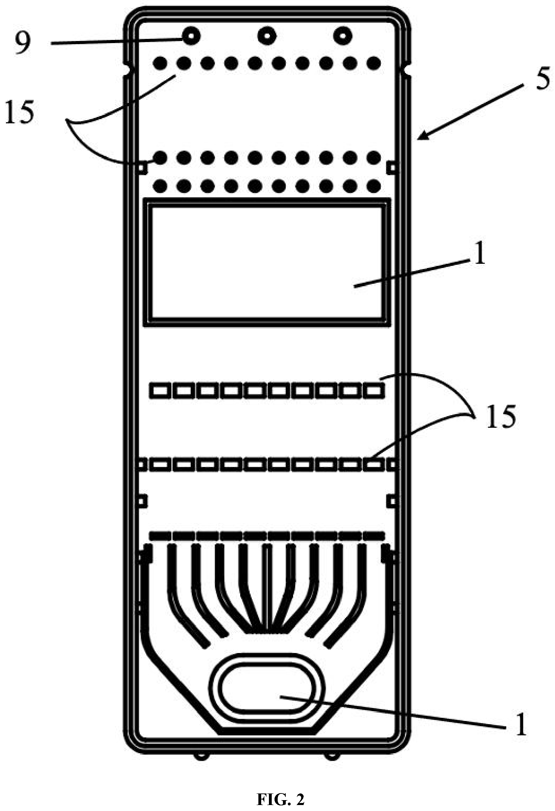

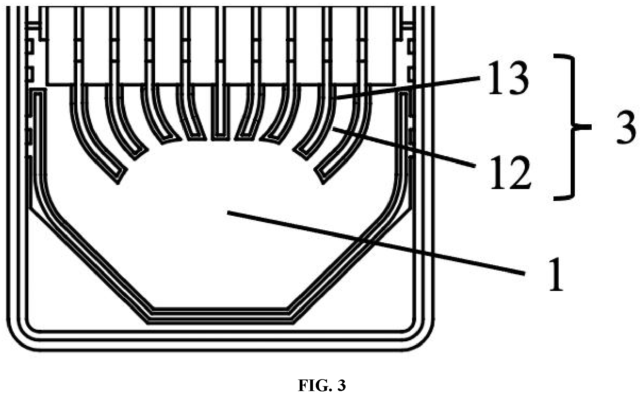

A hazard factor test cassette, which is a hazard factor test cassette based on bio-probe multi-channel immunochromatography. As shown in FIG. 1 and FIG. 2, it includes:
An outer shell, wherein the outer shell includes an upper shell (5) and a lower shell (6) assembled and connected with each other;
A sample injection port (1) for loading a sample;
Flow guide platforms (2), the number of the flow guide platforms (2) being 10, and the flow guide platforms (2) serving as detection channels for receiving individual detection test strips;
Flow guide paths, the number of the flow guide paths being 10, each of the flow guide paths independently communicating with the sample injection port (1) and one of the flow guide platforms (2), respectively; the flow guide paths including long-distance flow guide paths (3) and short-distance flow guide paths (4); the length of the connection between the sample injection port (1) and the flow guide platforms (2) via the long-distance flow guide paths (3) is greater than that via the short-distance flow guide paths (4); the detection test strips for detecting small-size toxin targets are disposed on the flow guide platforms (2) corresponding to the long-distance flow guide paths (3); the detection test strips for detecting larger-size bacterial targets are disposed on the flow guide platforms (2) corresponding to the short-distance flow guide paths (4); and the detection test strips for detecting viral targets with a size between the two are disposed on the flow guide platforms (2) corresponding to the flow guide paths between the long-distance flow guide paths (3) and the short-distance flow guide paths (4). As shown in FIG. 1 and FIG. 3, the flow guide paths include flow-guide grooves (7) disposed on the lower shell (6) and flow-guide sheets (8) disposed on the upper shell (5); in an assembled state of the outer shell, the flow-guide sheets (8) are inserted and fixed in the flow-guide grooves (7). The cooperatively inserted and connected flow-guide grooves (7) and flow-guide sheets (8) effectively prevent the sample solution from flowing out through the gaps between different flow guide paths between the upper shell and the lower shell. This not only can effectively block the cross-channel flow of the sample solution between different flow guide paths and the flow disturbance caused by the reflux of the sample solution, ensure the uniform distribution of the sample treatment solution in different flow guide paths, and guarantee the correctness and sensitivity of the detection results of different channels, but also the aforementioned flow-guide grooves (7) and flow-guide sheets (8) can effectively fix the upper shell (5) and the lower shell (6) of the test cassette together. Therefore, the test cassette provided by the present application does not need to be provided with a clamping structure for fixing the upper shell and the lower shell together near the flow guide paths, thereby reducing the size of the test cassette. Furthermore, to further improve the connection reliability of the flow-guide sheets (8) and the flow-guide grooves (7) and reduce the gap between them, the flow-guide sheets (8) and the flow-guide grooves (7) are inserted and connected in an interference fit manner;
A wide-vision detection window (11), wherein the wide-vision detection window (11) is used for observing the chromatographic states and final detection results of the detection test strips; the number of the wide-vision detection window (11) on the outer shell is one, and the wide-vision detection window (11) is disposed corresponding to the central axis of the sample injection port (1); both the long-distance flow guide paths (3) and the short-distance flow guide paths (4) are disposed opposite to the sample injection port (1);

Baffles (14) and fixing columns (15), wherein the baffles (14) are disposed on the lower shell (6) for separating adjacent detection test strips; the fixing columns (15) are disposed on the upper shell (5) for positioning the detection test strips.

In the present embodiment, as shown in FIG. 1 and FIG. 2, to firmly and reliably fix the upper shell (5) and the lower shell (6) together, an annular groove structure (10) is disposed on the lower shell (6) in the present application, and a rivet structure (9) is disposed on the upper shell (5) correspondingly. The aforementioned rivet structure (9) and annular groove structure (10) are inserted and connected in a matching manner. Moreover, since the flow guide paths in the present application are assembled and connected through the aforementioned flow-guide grooves (7) and flow-guide sheets (8), the flow-guide grooves (7) and flow-guide sheets (8) can firmly and reliably fix the lower shell (6) and the upper shell (5) together. Therefore, the aforementioned rivet structure (9) and annular groove structure (10) do not need to be disposed around the flow guide paths. It is only necessary to dispose the cooperatively connected rivet structure (9) and annular groove structure (10) on the side of the outer shell away from the flow guide paths.

As shown in FIG. 1, which is a three-dimensional structural schematic diagram of the lower shell with one wide-vision detection window; the 10 flow guide paths are arranged along the width direction of the outer shell; the sample injection port (1) is an elliptical structure extending along the width direction of the outer shell, and each of the long-distance flow guide paths (3) and the short-distance flow guide paths (4) is provided with a smooth curved section (12) extending toward the sample injection port (1) and a connecting section (13) extending toward the flow guide platforms (2).

Since bacteria, viruses, and toxins are in a dispersed and suspended state in the sample solution, their chromatography process is affected by the pore size of chromatographic media (such as flow-guide pads, sample pads, conjugate pads, and analytical membranes on the detection test strips) and their own resistance. The larger the size, the greater the resistance and the longer the chromatography time. Under the same chromatography time, the actual reaction time of large-size targets is relatively short. To ensure the synchronous chromatography of the targets to be detected on the detection test strips of each channel in the multi-channel test cassette, the channels for the detection test strips are assigned according to the inherent characteristics of the targets and the characteristics of the flow-guide structure. For example, compared with 2µm bacteria, 50nm toxins encounter different resistance due to size differences, resulting in faster chromatography speed of 50nm toxin targets and slower chromatography speed of 2µm bacterial targets. With the support of the flow-guide structure, the synchronization of chromatography of targets with different sizes to each test strip is achieved within the same chromatography time. Therefore, the 50nm toxin targets are assigned to the flow guide paths L1 to L3 and L8 to L10, which are far from the sample loading point as shown in FIG. 1, and the detection test strips for 2µm bacterial targets are assigned to the flow guide paths L4 to L7, which are close to the sample loading point as shown in FIG. 1. The above arrangement can effectively ensure the detection sensitivity of each detection target on the multi-channel test cassette.

In the present embodiment, when viruses also need to be detected, to avoid loss of detection sensitivity while achieving multi-channel detection, according to the above innovative structure and the size and movement principle of the analytes, the detection test strips for viruses (20nm-200nm) are assigned to the flow guide paths L3 and L8 as shown in FIG. 1, the toxin-based test strips (1nm-100nm) are assigned to the flow guide paths L1 to L2 and L9 to L10, and the bacterial detection test strips (0.5µm-5µm) are assigned to the detection channels L4 to L7. Among them, since anthrax spores have a relatively large size of 5µm-10µm, it is recommended that the detection test strips for anthrax spores be placed in the detection channels L5 and L6.

A hazard factor detection method, which includes the following steps:
Step S1: preparing detection test strips for virulent biological hazard factors and preparing a universal sample treatment solution;
Step S2: placing the detection test strips for identifying different types of the virulent biological hazard factors on different flow guide platforms 2 of a multi-channel test cassette, placing a flow-guide pad at a sample loading position, and covering and pressing an upper cover tightly;
Step S3: adding 1 mL to 3 mL of a sample prepared with the universal sample treatment solution into the sample injection port 1, recording a chromatographic time to determine a detection time;
Step S4: inserting a test strip end of a test cassette into a detector to a fixed position;
Step S5: starting the detector to perform quantitative determination on the test strips through the wide-vision detection window 11, and the detector displays a determination result;
Step S6: adding a fully negative sample into the sample injection port (1) of the test cassette, and reading a result on the detector, wherein each of the detection test strips is provided with an obvious quality control peak and an extremely low detection peak, and a ratio of the detection peak value to the quality control peak value presents a negative result;
Step S7: adding a to-be-detected sample prepared with the universal sample treatment solution into the sample injection port (1) of the test cassette, reading the result on the detector, and determining a result of a certain virulent biological hazard factor in the sample by regressing the ratio of the detection peak value to the quality control peak value with a standard curve built in the detector;
Wherein, in Step S2:
disposing the detection test strips for detecting large-size targets on the short-distance flow guide paths 4, and disposing the detection test strips for detecting small-size targets on the long-distance flow guide paths 3;
wherein, toxin-based detection test strips, virus-based detection test strips and bacteria-based detection test strips are sequentially disposed on the channels from long distances to short distances among the short-distance flow guide paths 4 and the long-distance flow guide paths 3.

Of course, the present embodiment does not specifically limit the arrangement positions of the flow-guide grooves 7 and flow-guide sheets 8 composing the flow guide paths. In other embodiments, the flow guide paths include: flow-guide grooves 7 disposed on the upper shell 5 and flow-guide sheets 8 disposed on the lower shell 6.

Of course, the present embodiment does not specifically limit the arrangement positions of the rivet structures 9 and annular groove structures 10. In other embodiments, to firmly and reliably fix the upper shell 5 and the lower shell 6 together, the rivet structures 9 are disposed on the upper shell 5 and the annular groove structures 10 are disposed on the lower shell 6 in the present application. The aforementioned rivet structures 9 and annular groove structures 10 are inserted and connected in a matching manner.

### Embodiment 2

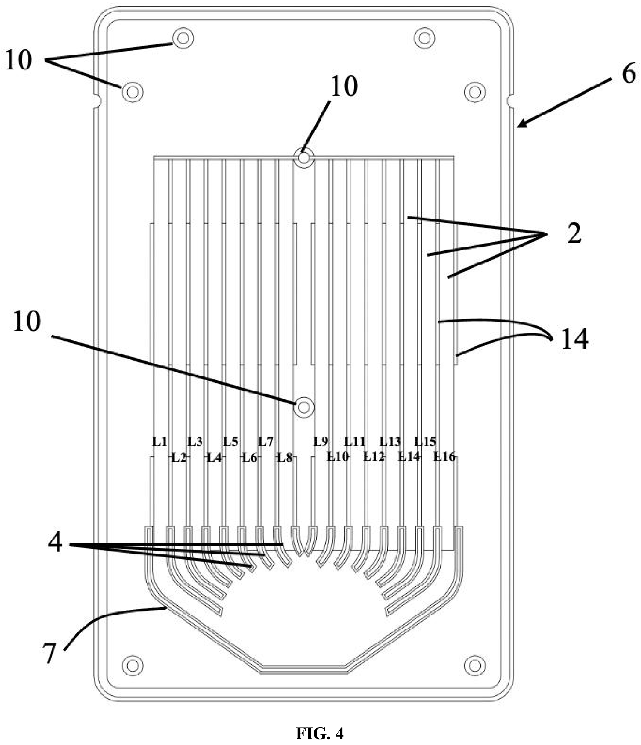

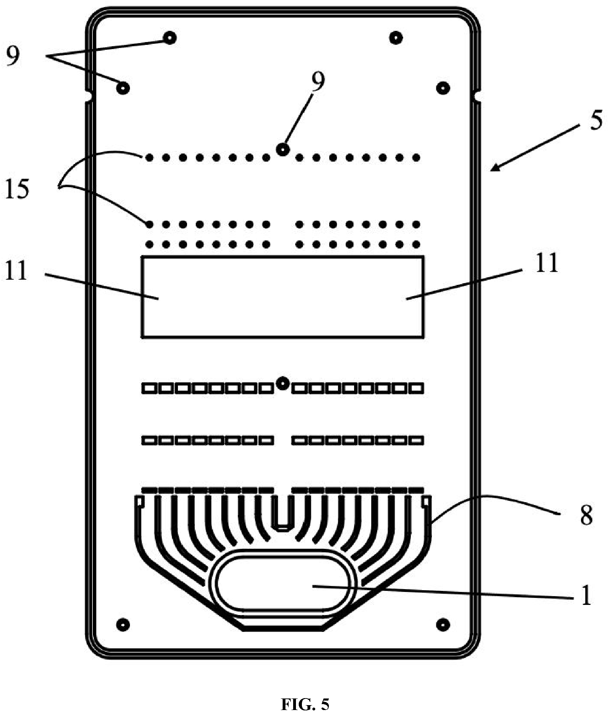

A hazard factor test cassette, which is a hazard factor test cassette based on bio-probe multi-channel immunochromatography. As shown in FIG. 4 and FIG. 5, it includes:
An outer shell, wherein the outer shell includes an upper shell 5 and a lower shell 6 assembled and connected with each other;
A sample injection port 1 for loading a sample;
Flow guide platforms 2, the number of the flow guide platforms 2 being 16, and the flow guide platforms 2 serving as detection channels for receiving individual detection test strips;
A wide-vision detection window 11, wherein the wide-vision detection window 11 is used for observing the chromatographic states and final detection results of the detection test strips; the number of the wide-vision detection window 11 on the outer shell is one, and the wide-vision detection window 11 is disposed corresponding to the central axis of the sample injection port 1; both the long-distance flow guide paths 3 and the short-distance flow guide paths 4 are disposed opposite to the sample injection port 1;
flow guide paths, the number of the flow guide paths being 16, each of the flow guide paths independently communicating with the sample injection port 1 and one of the flow guide platforms 2, respectively; the flow guide paths including long-distance flow guide paths 3 and short-distance flow guide paths 4; the length of the connection between the sample injection port 1 and the flow guide platforms 2 via the long-distance flow guide paths 3 is greater than that via the short-distance flow guide paths 4.

In the present embodiment, to avoid loss of detection sensitivity while achieving multi-channel detection, according to the above innovative structure and the size and movement principle of the analytes, the toxin-based detection test strips (1nm-100nm) are assigned to the detection channels L1 to L3, L14 to L16, and L7 to L10 as shown in FIG. 4; the virus-based test strips (20nm-200nm) are assigned to the detection channels L4, L5, L12, and L13; and the bacterial detection test strips (0.5µm-5µm) are assigned to the detection channels L6 to L11. Among them, since anthrax spores have a relatively large size of 5µm-10µm, it is recommended that the detection test strips for anthrax spores be placed in the detection channels L8 and L9.

Obviously, the above embodiments are merely examples for clear illustration, and are not intended to limit the implementation modes. For those of ordinary skill in the art, other different forms of changes or modifications can be made on the basis of the above description. It is unnecessary and impossible to enumerate all the implementation modes here. However, the obvious changes or modifications derived therefrom still fall within the protection scope of the present invention.

## Claims

1. A hazard factor test cassette, which is a hazard factor test cassette based on bio-probe multi-channel immunochromatography, **characterized in that** the hazard factor test cassette comprises:
an outer shell;
a sample injection port (1) for loading a sample;
flow guide platforms (2), a number of the flow guide platforms (2) being 2 to 20, and the flow guide platforms (2) being configured to receive individual detection test strips; and
flow guide paths, a number of the flow guide paths being 2 to 20, each of the flow guide paths and a flow-guide pad independently communicating with the sample injection port (1) and one of the flow guide platforms (2), respectively; the flow guide paths comprising: long-distance flow guide paths (3) and short-distance flow guide paths (4); a length of connection between the sample injection port (1) and the flow guide platforms (2) via the long-distance flow guide paths (3) being greater than that via the short-distance flow guide paths (4); the detection test strips for detecting small-size targets are disposed on the flow guide platforms (2) corresponding to the long-distance flow guide paths (3), and the detection test strips for detecting large-size targets are disposed on the flow guide platforms (2) corresponding to the short-distance flow guide paths (4).

2. The hazard factor test cassette according to claim 1, **characterized in that** the outer shell comprises: an upper shell (5) and a lower shell (6) assembled and connected with each other;
the flow guide paths comprise: flow-guide grooves (7) disposed on one of the upper shell (5) and the lower shell (6), and flow-guide sheets (8) disposed on the other of the upper shell (5) and the lower shell (6); and
in an assembled state of the outer shell, the flow-guide sheets (8) are inserted and fixed in the flow-guide grooves (7).

3. The hazard factor test cassette according to claim 2, **characterized in that** the flow-guide sheets (8) and the flow-guide grooves (7) are inserted and connected in an interference fit manner.

4. The hazard factor test cassette according to claim 2, **characterized in that** the hazard factor test cassette further comprising: rivet structures (9) disposed on one of the upper shell (5) and the lower shell (6), and annular groove structures (10) disposed on the other of the upper shell (5) and the lower shell (6); and
the rivet structures (9) and the annular groove structures (10) are inserted and connected in a matching manner to fix the upper shell (5) and the lower shell (6) together.

5. The hazard factor test cassette according to claim 4, **characterized in that** the rivet structures (9) and the annular groove structures (10) that are connected in the matching manner are disposed on a side of the outer shell away from the flow guide paths.

6. The hazard factor test cassette according to any one of claims 2 to 5, **characterized in that** a plurality of the flow guide paths are disposed in a width direction of the outer shell;
the sample injection port (1) is an elliptical structure extending along the width direction of the outer shell, and each of the long-distance flow guide paths (3) and/or the short-distance flow guide paths (4) is provided with: a smooth curved section (12) extending toward the sample injection port (1), and a connecting section (13) extending toward the flow guide platforms (2).

7. The hazard factor test cassette according to claim 6, **characterized in that** the hazard factor test cassette further comprising:
a wide-vision detection window (11) being configured to observe chromatographic states and final detection results of the detection test strips.

8. The hazard factor test cassette according to claim 7, **characterized in that** a number of the wide-vision detection window (11) on the outer shell is one, the wide-vision detection window (11) is disposed corresponding to a central axis of the sample injection port (1); the long-distance flow guide paths (3) and the short-distance flow guide paths (4) are both disposed opposite to the sample injection port (1); or,
a number of the wide-vision detection windows (11) on the outer shell is two, the two wide-vision detection windows (11) are symmetrically disposed on both sides of the sample injection port (1); and the long-distance flow guide paths (3) and the short-distance flow guide paths (4) are disposed on both sides of the sample injection port (1).

9. The hazard factor test cassette according to claim 2, **characterized in that** the lower shell (6) is provided with baffles (14) for separating the detection test strips, and the upper shell (5) is further provided with fixing columns (15) for positioning the detection test strips.

10. A hazard factor test method for using the hazard factor test cassette according to any one of claims 1 to 9, **characterized in that** the method comprises the following steps:
step S1: preparing detection test strips for virulent biological hazard factors and preparing a universal sample treatment solution;
step S2: placing the detection test strips for identifying different types of the virulent biological hazard factors on different flow guide platforms (2) of a multi-channel test cassette, placing a flow-guide pad at a sample loading position, and covering and pressing an upper cover tightly;
step S3: adding 1 mL to 3 mL of a sample prepared with the universal sample treatment solution into the sample injection port (1), recording a chromatographic time to determine a detection time;
step S4: inserting a test strip end of a test cassette into a detector to a fixed position;
step S5: starting the detector to perform quantitative determination on the test strips through the wide-vision detection window (11), and the detector displays a determination result;
step S6: adding a fully negative sample into the sample injection port (1) of the test cassette, and reading a result on the detector, wherein each of the detection test strips is provided with an obvious quality control peak and an extremely low detection peak, and a ratio of the detection peak value to the quality control peak value presents a negative result; and
step S7: adding a to-be-detected sample prepared with the universal sample treatment solution into the sample injection port (1) of the test cassette, reading the result on the detector, and determining a result of a certain virulent biological hazard factor in the sample by regressing the ratio of the detection peak value to the quality control peak value with a standard curve built in the detector;
wherein, in step S2:
disposing the detection test strips for detecting large-size targets on the short-distance flow guide paths (4), and disposing the detection test strips for detecting small-size targets on the long-distance flow guide paths (3);
wherein, toxin-based detection test strips, virus-based detection test strips and bacteria-based detection test strips are sequentially disposed on the channels from long distances to short distances among the short-distance flow guide paths (4) and the long-distance flow guide paths (3).
